# EUROPÄISCHE PATENTSCHRIFT

(11) **EP 0 563 080 B1**
(45) Veröffentlichungstag und Bekanntmachung des Hinweises auf die Patenterteilung: **15.07.1998**
(21) Anmeldenummer: 92900175.8
(22) Anmeldetag: 09.12.1991
(51) Int. Cl.: G01N 33/34, G01N 15/14, G01N 21/64

(54) **MESSVERFAHREN ZUR BESTIMMUNG VON HARZTEILCHEN IN PAPIERSTOFFEN**
MEASURING METHOD FOR RESIN PARTICLES IN PAPER PULP
PROCEDE DE MESURE DE PARTICULES DE RESINES PRESENTES DANS DES PATES A PAPIER

(30) Priorität: 18.12.1990 DE 4040463
(43) Veröffentlichungstag der Anmeldung: 06.10.1993
(73) Patentinhaber: BASF Aktiengesellschaft, 67063 Ludwigshafen (DE)
(72) Erfinder: HORN, Dieter, D-6900 Heidelberg (DE); LUEDDECKE, Erik, D-6704 Mutterstadt (DE); GIERULSKI, Alfred, D-6900 Heidelberg-Ziegelhausen (DE); KROEHL, Thomas, D-6500 Mainz 1 (DE); LORENCAK, Primoz, D-6700 Ludwigshafen (DE)
(86) Internationale Anmeldenummer: EP9102350
(87) Internationale Veröffentlichungsnummer: WO9211534

(56) Entgegenhaltungen:
- EP-A- 347 696
- CA-A- 1 136 032
- US-A- 4 837 446
- MEDICAL PHYSICS. Bd. 7, Nr. 6, November 1980, NEW YORK US Seiten 609 - 615; A. NORMAN: 'Flow cytometry'

## Beschreibung

Die Erfindung betrifft ein Meßverfahren zur Bestimmung der Anzahl und Größe von in Papierstoff frei verteilten Harzteilchen.

Bei der Papierherstellung auf pH-neutral.er Verfahrensweise treten Probleme durch die im Papierstoff vorhandenen natürlichen Harzteilchen auf. Insbesondere die freien, d. h. nicht an die Holzfasern gebundenen Harzpartikel führen durch Ablagerungen in den Papiermaschinen zu erheblichen Betriebsstörungen z. B. Papierabrissen, und folglich zu teueren Ausfallzeiten. Daher werden dem Papierstoff Hilfsmittel zugesetzt, die die Harzteilchen an die Holzfasern binden und so den Austrag des Harzes mit dem Papier aus der Papiermaschine gewährleisten. Die Wirksamkeit solcher Hilfsmittel wurde bisher durch aufwendige und wenig zuverlässige Versuche an den Papiermaschinen selbst getestet. Ferner werden in der Literatur Methoden beschrieben, die eine Bestimmung der schädlichen Harzmenge im Labor gestatten. Der Nachweis freier Harzpartikel und die Prüfung der Wirksamkeit der eingesetzten Hilfsmittel ist im Labormaßstab jedoch insofern stark erschwert, als das ungebundene Harz nur in sehr geringen Mengen im Papierstoff vorliegt (etwa 1 g freies Harz auf 1 t Papierstoff). Daher führen die bekannten, herkömmlichen Bestimmungsmethoden für schädliches Harz, wie die Extraktion des Papierstoffes mit organischen Lösungsmitteln, z. B. mit Dichlormethan (Weigl et al., "Das Papier", Band 40, 1986, Seite V52), die Abscheidung des Harzes an Oberflächen z. B. nach Gustafson (Gustafson C. et al., "Papieri ja Puu", Band 34, 1952, Seiten 121 bis 127), die Foltationsmethode nach Störle und Teves (Störle und Teves, "Das Papier", Band 10, 1956, Seiten 264 bis 270) und die Methode der mikroskopischen Auszählung (Allen, L.H., "Pulp & Papier", Canada, Band 76, 1975, Seite 70) nur zu unbefriedigenden und umstrittenen Ergebnissen.

CA-A-1 136 032 beschreibt ein Verfahren zur Bestimmung von Harzteilchen in Papierstoff, wobei die Harzteilchen durch Filtration abgetrennt werden und dann mittels eines aus der Medizin bekannten Zytometers gezählt werden.

In Medical Physics., Bd. 7, Nr. 6, Nov. 1980, New York US, Seiten 609 - 615 wird ein aus der Medizin bekanntes Durchflußzytometer offenbart; die Teilchen werden mit einem Fluoreszenzfarbstoff markiert, mittels Hüllstromküvette vereinzelt und mittels Laser und Vierkanalanalyse detektiert.

In der US-A-4 837 446 wird eine Vorrichtung und ein Verfahren zur Ermittlung der Gleichmäßigkeit einer Paierstoffsuspension offenbart.

Die EP-A 0 347 696 beschreibt eine Anordnung zur Messung des Dispergiergrades in strömenden Suspensionen.

Vorliegender Erfindung liegt die Aufgabe zugrunde, ein Meßverfahren zur Bestimmung der Anzahl und Größe von in Papierstoff frei verteilten Harzteilchen zu entwickeln, das ohne großen Aufwand auch bei sehr niedriger Konzentration an dispers verteiltem Harz im Papierstoff zuverlässig durchführbar ist.

Zur Lösung der Aufgabe werden beim Mepverfahren nach der Erfindung zunächst eine Papierstoffsuspension hergestellt und davon die Harzteilchen durch Filtration abgetrennt, sodann werden die Harzteilchen mit einem Fluoreszenzfarbstoff markiert und nach Vereinzelung zur Lichtemission angeregt, die Lichtsignale der einzelnen Harzteilchen detektiert und die Detektionssignale zur Zählung und Größenbestimmung der Harzteilchen ausgewertet.

Das Mepverfahren ist an einem Ausführungsbeispiel anhand der Zeichnung nachfolgend im einzelnen beschrieben.

Es zeigen
- Figur 1: eine Meßzelle zur Vereinzelung der Harzteilchen in einem schematischen Längsschnitt
- Figur 2: eine Meßanordnung zur Durchführung des Meßverfahrens
- Figur 3: ein Diagramm der Abhängigkeit der Anzahl der Harzteilchen von der Hilfsmittelkonzentration

Nach Herstellung einer Suspension aus dem zu untersuchenden Papierstoff und Wasser wird eine Filtration der Suspension zur Abtrennung der Harzteilchen vom Papierstoff vorgenommen.

Dies geschieht mit einem sog. Dynamic Drainage Jar, einem Plexiglasbehälter mit eingebautem Papiersieb der Maschenweite 80 µm, indem der zu untersuchende Papierbrei zuerst auf etwa 0.4 % Feststoffgehalt (Praxiswert) verdünnt und unter Rühren über das Sieb abfiltriert wird. Das Hilfsmittel zur Bindung der Harzteilchen an die Holzfasern wird der verdünnten Papierstoffsuspension vor der Filtration in einer Konzentration zwischen 0 und 0.5 %, bezogen auf den Faser-Feststoffgehalt, zugesetzt. Das gewonnene Filtrat, welches Harzteilchen und Fasern mit einer Gröpe kleiner als 80 µm enthält, wird nochmals verdünnt (1:10) und im Verhältnis 1:25 mit einer Farbstofflösung, vorzugsweise mit dem Fluoreszenzfarbstoff Fluorol® 555 der Firma BASF AG (N-(n-butyl)-4-(n-butylamino)-Naphthalsäureimid) in Lösung mit 40 mg/l Ethanol, versetzt. Dabei werden nur die Harzteilchen gefärbt, nicht jedoch die Holzfasern. Nach einer Färbezeit von ca. 2 Minuten kann mit der eigentlichen Messung begonnen werden.

Die Bestimmung der Anzahl und Gröpe der Harzteilchen kann an vereinzelten, strömenden Teilchen unter Anwendung sowohl eines kohärenten, elastischen, winkelabhängigen Lichtstreuprozesses als auch des inkohärenten, winkelunabhängigen Streuphänomens der Fluoreszenz erfolgen. In beiden Fällen werden die Teilchen einzeln von einem Laserstrahl beleuchtet.

Die Vereinzelung der Harzteilchen geschieht nach dem Prinzip der hydrodynamischen Fokussierung, indem die Teilchensuspension in einer Hüllstromküvette in einen Hüllstrom von fließendem Wasser eingeleitet und durch die relativ zum Probenstrom wesentlich höhere Strömungsgeschwindigkeit des Hüllstroms verdünnt wird und die Teilchen in der Mitte des gemeinsamen Wasserstrahls hydrodynamisch fokussiert und anschließend durch einen fokussierten Laserstrahl hindurchgeführt werden.

Zur Anregung der zumeist im blauen Wellenlängenbereich absorbierenden Fluoreszenzfarbstoffe wird die am besten zum Absorptionsmaximum passende Linie eines Lasers verwendet. Das zumeist gelbe Fluoreszenzlicht (um 550 nm) wird über ein Kantenfilter und eine Linse unter einem Beobachtungswinkel von 90° auf einen Detektor, beispielsweise einen Photomultiplier, abgebildet. Das Kantenfilter schirmt den Photomultiplier vor dem zur Anregung verwendeten blauen Laser-Streulicht ab.

Die Einzelimpulse des Photomultipliers werden verstärkt und einem Vielkanalanalysator zugeführt. Dort werden die Impulse nach ihrer Intensität sortiert, gezählt und in einem Impulshöhendiagramm dargestellt. Zur Einstellung der Apparatur und Kontrolle der Messung werden die verstärkten Impulse gleichzeitig einem Oszilloskop zugeleitet. Die Zähldauer pro Messung beträgt 100 s. Der Vielkanalanalysator ist in einen Personalcomputer integriert, in dem die aufgenommenen Daten gespeichert und ausgewertet werden.

Die Fluoreszenzintensität ist proportional zum Volumen der Teilchen, da man annehmen kann, daß die Zahl der Fluoreszenzmoleküle pro Einheitsvolumen für jedes Teilchen konstant ist. Aus der Impulshöhe kann somit auf die Gröpe der gezählten Harzteilchen geschlossen werden. Die Kalibrierung der Apparatur erfolgt mit fluoreszenzmarkierten Eichlatizes bekannter Größe.

Mit Hilfe des Meßverfahrens nach der Erfindung können Impulshöhenverteilungen von Papierstoffiltraten mit und ohne Hilfsmittelzusatz aufgenommen und die jeweilige Harzteilchenzahl als Summe der Kanalinhalte bestimmt werden. Als Maß für die Wirksamkeit der eingesetzten Hilfsmittel wird die prozentuale Abnahme der Harzteilchenzahl nach Zusatz des Hilfsmittels herangezogen.

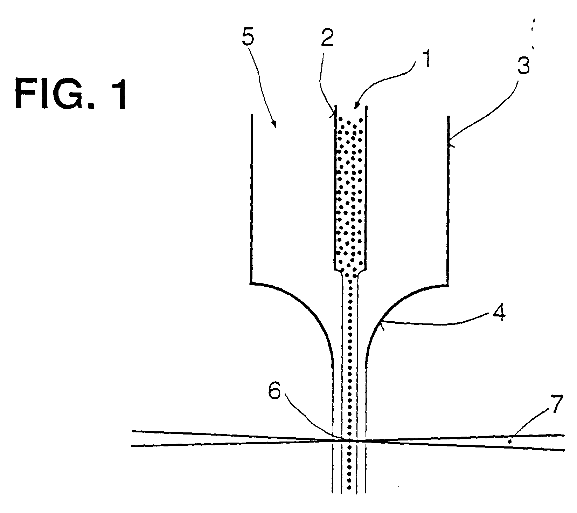

Der Aufbau der Meßzelle zur Vereinzelung und Detektion der Harzteilchen ist in Figur 1 schematisch dargestellt. Das Papierstoffiltrat 1 wird durch eine Kapillare 2 (∅ = 100 µm) in eine Hüllstromküvette 3 geleitet, an deren Ende sich eine 200 µm weite Düse 4 befindet. Die gegenüber dem Probenstrom 1 höhere Strömungsgeschwindigkeit des Hüllstromes 5 (Verhältnis 100:1) verdünnt die Teilchensuspension und führt zu einer hydrodynamischen Fokussierung der Harzteilchen im Zentrum des gemeinsamen Flüssigkeitsstrahles. Die Harzpartikel werden somit in einem freifallenden Strahl einzeln durch den Meport 6 geführt, der mit dem Fokus des Laserstrahles 7 übereinstimmt.

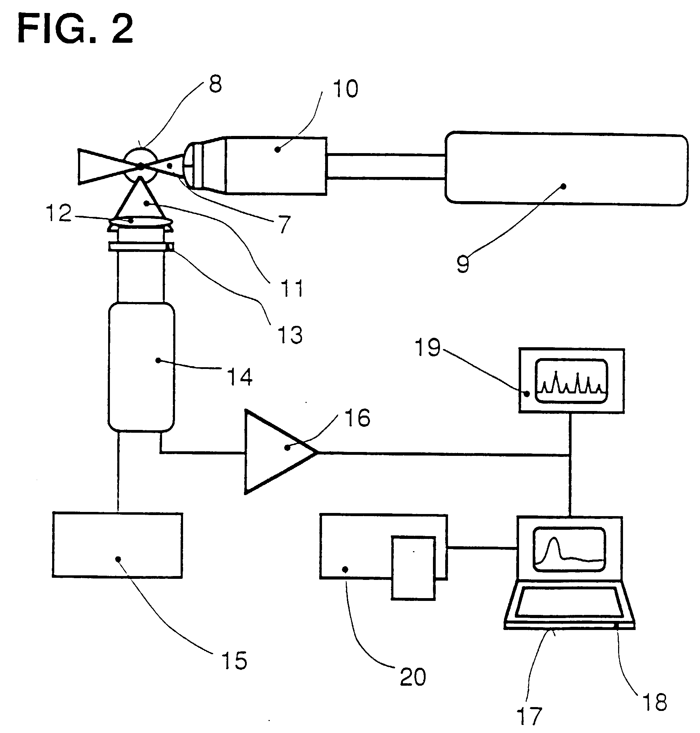

Die komplette Meßanordnung, in die die Meßzelle aus Figur 1 integriert ist, ist in Figur 2 gezeigt. Die durch hydrodynamische Fokussierung vereinzelten Harzteilchen laufen in der Meßzelle 8 durch den Brennpunkt des Laserstrahles 7, der von einem Laser 9 über ein Objektiv 10 in das Zentrum der Meßzelle fokussiert wird. Das von den markierten Harzteilchen emittierte Fluoreszenzlicht 11 wird senkrecht zum anregenden Strahl und senkrecht zum Probenstrom über eine Linse 12 und ein Kantenfilter 13 auf einen Photomultiplier 14 abgebildet, der von dem Spannungsgerät 15 mit Hochspannung versorgt wird. Die elektrischen Signale des Photomultipliers werden in einem Verstärker 16 logarithmisch verstärkt und anschließend einem Vielkanalanalysator 17, der in einen Personalcomputer 18 integriert ist, zugeführt. Zur Einstellung der Apparatur und Kontrolle der Messung werden die Signale gleichzeitig auf einem Oszillographen 19 dargestellt. Die aufgenommenen Impulshöhendiagramme und die ausgewerteten Meßergebnisse können auf einem Plotter/Drucker 20 ausgegeben werden.

Die erfindungsgemäß eingesetzte Apparatur kann in der Praxis zur Prozeßkontrolle bei der Herstellung von Papier hinsichtlich schädlichen Harzes im Papierstoff eingesetzt werden. Mit der Information über den Anteil schädlichen Harzes, die durch das Meßverfahren nach der Erfindung erhalten wird, kann die Zufuhr von Hilfsmitteln zum Papierstoffauflauf gesteuert werden.

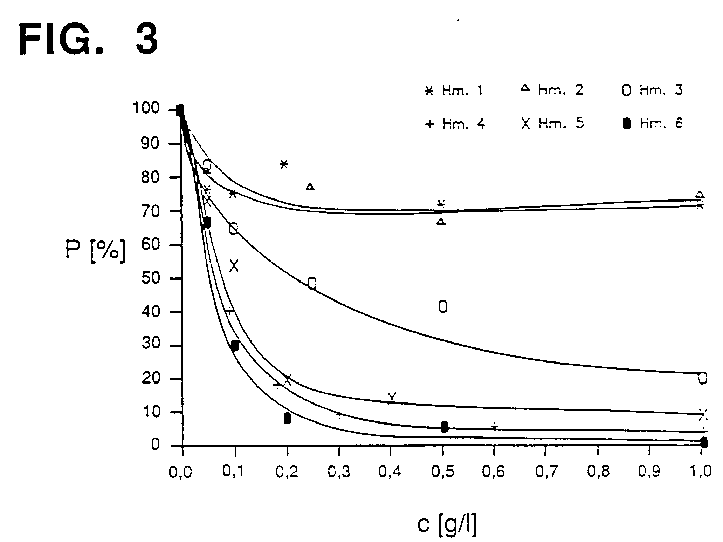

In Figur 3 ist ein Diagramm der Ergebnisse von Messungen bei verschiedenen Hilfsmittelkonzentrationen (Hm1-Hm6) dargestellt. Aufgetragen ist der prozentuale Anteil P [%] der noch im Filtrat vorhandenen Harzteilchen in Abhängigkeit der Hilfsmittelkonzentration C [g/l]. Die Meßpunkte stellen jeweils das Flächenverhältnis der Verteilungskurve mit Hilfsmittel zu der Kurve ohne Hilfsmittel dar. Die Abbildung zeigt neben der Abnahme der Harzteilchenzahl für jedes Hilfsmittel auch deren unterschiedliche Wirksamkeit.

Zur Anfärbung der dispers verteilten Harzteilchen wurde der Fluoreszenzfarbstoff Fluorol 555 (N-(n-butyl)-4-(n-butylamino)-Naphthalsäureimid) verwendet. Das Absorptionsmaximum dieses Farbstoffes liegt bei 440 nm. Zur Anregung bietet sich daher vorzugsweise die 442-nm-Linie eines He-Cd-Lasers an. Bei Verwendung anderer Fluoreszenzfarbstoffe ist der Laser und das Kantenfilter entsprechend anzupassen.

## Patentansprüche

1. Meßverfahren zur Bestimmung der Anzahl und Größe der Harzteilchen, die nach der Filtration einer Papierstoffsuspension im Filtrat gezählt werden, dadurch gekennzeichnet, daß die Harzteilchen mit einem Fluoreszenzfarbstoff markiert und nach Vereinzelung zur Lichtemission angeregt werden, die Lichtsignale der einzelnen Harzteilchen detektiert und die Detektionssignale zur Zählung und Größenbestimmung der Harzteilchen ausgewertet werden.

2. Mepverfahren nach Anspruch 1, dadurch gekennzeichnet, daß die Harzteilchen mit Hilfe einer Hüllstromküvette durch hydrodynamische Fokussierung vereinzelt werden.

3. Mepverfahren nach Anspruch 1 und 2, dadurch gekennzeichnet, daß die Harzteilchen zur Anregung der Lichtemission durch den Fokus eines Laserstrahls geführt werden.

4. Mepverfahren nach Anspruch 3, dadurch gekennzeichnet, daß Laserlicht eingesetzt wird, dessen Wellenlänge mit der Absorptionsbande des zur Markierung verwendeten Fluoreszenzfarbstoffs übereinstimmt.

5. Meßverfahren nach Anspruch 1 bis 4, dadurch gekennzeichnet, daß als Fluoreszenzfarbstoff N-(n-butyl)-4-(n-butylamino)-Naphthalsäureimid verwendet wird.

6. Mepverfahren nach Anspruch 1 bis 5, dadurch gekennzeichnet, dap die Lichtsignale der Harzteilchen mit Hilfe eines Photomultipliers detektiert werden, dessen Signale in einem Vielkanalanalysator ausgewertet werden.

## Claims

1. A method for determining the number and size of resin particles which are counted in the filtrate after the filtration of a paper stock suspension, wherein the resin particles are marked with a fluorescent dye, isolated and then excited to produce light emission, the light signals of the individual resin particles are detected and the detection signals are evaluated for counting and size determination of the resin particles.

2. A method as claimed in claim 1, wherein the resin particles are isolated by hydrodynamic focusing with the aid of an envelope stream cell.

3. A method as claimed in claims 1 and 2, wherein the resin particles are passed through the focus of a laser beam for excitation of light emission.

4. A method as claimed in claim 3, wherein laser light whose wavelength corresponds to the absorption band of the fluorescent dye used for marking is employed.

5. A method as claimed in any of claims 1 to 4, wherein the fluorescent dye used is N-(n-butyl)-4-(n-butylamino)-naphthalimide.

6. A method as claimed in any of claims 1 to 5, wherein the light signals of the resin particles are detected with the aid of a photomultiplier, whose signals are evaluated in a multichannel analyzer.

## Revendications

1. Procédé de mesure pour la détermination du nombre et de la taille de particules de résine, compté après filtration d'une suspension de pâte de papier dans un filtrat, caractérisé par le fait que les particules de résine sont marquées avec un colorant à fluorescence et sont excitées en émission lumineuse après individualisation, les signaux lumineux des différentes particules de résine sont détectés et les signaux de détection sont exploités pour le comptage et la détermination de la taille des particules de résine.

2. Procédé de mesure selon la revendication 1, caractérisé par le fait que les particules de résine sont individualisées à l'aide d'une cuvette à écoulement en enveloppe par une focalisation hydrodynamique.

3. Procédé de mesure selon les revendications 1 et 2, caractérisé par le fait que les particules de résine sont guidées par le foyer d'un rayon laser pour produire l'excitation de l'émission lumineuse.

4. Procédé de mesure selon la revendication 3, caractérisé par le fait qu'est utilisée une lumière laser dont la longueur d'onde coïncide avec la bande d'absorption du colorant à fluorescence utilisé pour le marquage.

5. Procédé de mesure selon les revendications 1 à 4, caractérisé par le fait qu'on utilise comme colorant à fluorescence du N-(n-butyle)-4-(n-butylamino)-acide naphtalique imide.

6. Procédé de mesure selon les revendications 1 à 5, caractérisé par le fait que les signaux lumineux des particules de résine sont détectés à l'aide d'un photomultiplicateur dont les signaux sont évalués dans un analyseur à plusieurs canaux.
